## Europäisches Patentamt
## European Patent Office
## Office européen des brevets

(11) Veröffentlichungsnummer: **0 060 918**
**A1**

(12) # EUROPÄISCHE PATENTANMELDUNG

(21) Anmeldenummer: **81108232.0**

(22) Anmeldetag: **12.10.81**

(51) Int. Cl.³: **G 01 N 21/90**

(30) Priorität: **11.03.81 DE 3109270**

(43) Veröffentlichungstag der Anmeldung:
**29.09.82 Patentblatt 82/39**

(84) Benannte Vertragsstaaten:
**DE FR GB NL**

(71) Anmelder: **SIEMENS AKTIENGESELLSCHAFT Berlin
und München
Postfach 22 02 61
D-8000 München 22(DE)**

(72) Erfinder: **Marguerre, Hans-Helmut, Dipl.-Phys.
Mähfeldstrasse 23
D-7541 Straubenhardt 4(DE)**

(54) **Anordnung zur Flascheninspektion.**

(57) Die Inspektion einer Flasche (1) auf Verunreinigungen und Beschädigungen soll mit einer einzigen Fernsehkamera (20) bzw. einer entsprechenden Diodenmatrix erfolgen. Hierzu ist vorgesehen, die Flasche von unten diffus zu beleuchten und zunächst die am Flaschenmund (2) austretenden Lichtstrahlen über eine Sammellinse (22) oder ein innenverspiegeltes Rohr (24) einer Fernsehkamera (20) oder Diodenmatrix zuzuführen. Die Anordnung weiterer optischer Linsen (26, 27) zwischen der Sammellinse und der Fernsehkamera oder der Diodenmatrix oder innerhalb des innenverspiegelten Rohres ermöglicht es, gleichzeitig den Flaschenboden (4) und die Innenwand (3) zu erfassen. Die Sammmellinse kann dazu mit einer zentrischen Bohrung (25) versehen sein. Bei genügend großem Durchmesser der Sammellinse oder des innenverspiegelten Rohres kann auch die Außenwand (3) der Flasche erfaßt werden.

FIG 7

FIG 8

EP 0 060 918 A1

SIEMENS AKTIENGESELLSCHAFT

Berlin und München

Unser Zeichen

VPA 81 P 3508

0060918

Anordnung zur Flascheninspektion

1. Anwendungsgebiet der Erfindung

Die Erfindung liegt auf dem Gebiet der meßtechnischen Erfassung von Oberflächenstrukturen an rotationssymmetrischen Körpern und ist insbesondere bei der Inspektion von Flaschen auf Verunreinigungen und Beschädigungen anzuwenden.

2. Stand der Technik

Bei der Inspektion von Flaschen auf Verunreinigungen und Beschädigungen sind die drei Bereiche Flaschenmund, Flaschenwand (innen und außen) und Flaschenboden zu unterscheiden. In aller Regel werden diese drei bzw. vier Bereiche getrennt erfaßt und ausgewertet. Zur Beobachtung des Flaschenmundes ist beispielsweise eine Anordnung bekannt, bei der der Flaschenmund mittels eines Umlenkspiegels von oben beleuchtet wird und bei dem die reflektierten Lichtstrahlen über eine optische Anordnung einer Fernsehkamera zugeführt werden, die mit einer elektronischen Auswerteeinrichtung verkoppelt ist (DE-OS 23 18 849).Bei einer anderen bekannten Anordnung erfolgt die Beleuchtung des Flaschenmundes mit einer konzentrisch zum Flaschenmund angeordneten torusförmigen Lichtquelle, während die reflektierten Lichtstrahlen über eine Optik einer ringförmigen Diodenmatrix zugeführt werden (DE-OS 27 18 802). Eine Variante dieser Anordnung besteht darin, den Flaschenmund mit Hilfe eines Ringes aus Leuchtdioden zu beleuchten und die reflektierten Lichtstrahlen über eine entsprechende Optik einer Fernsehkamera zuzuführen (DE-OS 29 16 361).

Es ist auch schon vorgeschlagen worden, Flaschenmund und Flaschenboden gleichzeitig zu beobachten. Hierzu sind für

0060918

jeden Bereich voneinander getrennte Lichtquellen und optische Einrichtungen vorgesehen, wobei die optische Einrichtung zur Beobachtung des Flaschenbodens in die Einrichtung zur Beobachtung des Flaschenmundes räumlich integriert sein kann. Die Beobachtung des Flaschenmundes erfolgt dabei mit Hilfe eines rotationssymmetrischen Spiegelsystems (DE-ANM P 30 35 082.4). Die Beobachtung des Flaschenbodens allein kann beispielsweise mit einer Anordnung erfolgen, bei der unterhalb des Flaschenbodens eine Beleuchtungseinrichtung angeordnet ist, wobei der Flaschenboden mit Hilfe einer über dem Flaschenmund angeordneten Fernsehkamera mit zugeordneter Optik durch den Flaschenhals hindurch beobachtet wird (DE-OS 28 48 316).

Um weiterhin die Wandung einer Flasche mit einer einzigen Fernsehkamera erfassen zu können, ist es bekannt, zwei Bilder der Flasche aus unterschiedlichen Blickrichtungen zu erzeugen und diese mittels eines Spiegelsystems nebeneinander auf die lichtempfindliche Schicht der Fernsehkamera zu projizieren (DE-PS 26 17 457).

3. Darstellung der Erfindung

  a) Technische Aufgabe

Der Erfindung liegt die Aufgabe zugrunde, zur Erfassung der Oberflächenstruktur eines rotationssymmetrischen Körpers, insbesondere zur Erfassung von Beschädigungen an einem Flaschenmund, eine Anordnung zu schaffen, die eine gleichzeitige Beobachtung weiterer Teile des rotationssymmetrischen Körpers, insbesondere eine gleichzeitige Beobachtung von Boden und Wand einer Flasche, unter Verwendung nur einer einzigen Lichtquelle ermöglicht. Insbesondere soll bei der Beobachtung eines Flaschenmundes gewährleistet sein, daß bei unverändertem Grundaufbau der Anordnung auch Flaschenboden und Flaschenwand beobachtet werden können.

b) Lösung

Ausgehend von einer Anordnung zur Erfassung der Oberflächenstruktur eines rotationssymmetrischen Körpers, insbesondere zur Erfassung von Beschädigungen an einem Flaschenmund, die aus einer Beleuchtungseinrichtung, aus einer optischen Einrichtung, einem lichtempfindlichen elektronischen Bauteil und einer diesem lichtempfindlichen elektronischen Bauteil nachgeordneten elektronischen Einrichtung zur Auswertung elektrischer Bildsignale besteht, wobei die optische Einrichtung die von dem Körper ausgehenden Lichtstrahlen um- oder ablenkt und auf das lichtempfindliche elektronische Bauteil projiziert, ist zur Lösung dieser Aufgabe erfindungsgemäß vorgesehen, daß die Beleuchtungseinrichtung auf der dem lichtempfindlichen elektronischen Bauteil abgekehrten Seite des rotationssymmetrischen Körpers angeordnet ist und daß die optische Einrichtung aus einer Sammellinse (Fresnel-Linse, Asphäre) oder aus einem innenverspiegelten Rohr besteht, deren bzw. dessen Durchmesser wesentlich größer ist als der Durchmesser des Körpers.

c) Vorteile

Für die derart ausgebildete Anordnung ist charakteristisch, daß die Beleuchtungseinrichtung nicht oberhalb oder konzentrisch zu dem zu prüfenden Körper, sondern auf der dem lichtempfindlichen elektronischen Bauteil abgekehrten Seite, also bei einer Flasche im Bereich des Flaschenbodens, angeordnet ist. Dies hat einerseits zur Folge, daß der jeweilige Körper, also insbesondere der Flaschenmund, nicht mittels Lichtstrahlen überprüft wird, die an der Oberfläche des Körpers reflektiert werden, sondern mit Lichtstrahlen, die aus der Oberfläche austreten. Andererseits wird dadurch die Voraussetzung für eine weitergehende Überprüfung des Körpers, bei einer Flasche also auch des Bodens und der Wand, geschaffen; denn diese Bereiche können ebenfalls durch die im Bodenbereich der Flasche angeordnete Beleuchtungseinrichtung ausgeleuchtet bzw. durchleuchtet werden.

Da die aus dem Körper austretenden Lichtstrahlen generell nicht in Richtung auf das lichtempfindliche elektronische Bauteil verlaufen, ist die Verwendung einer Sammellinse in Form einer Fresnel-Linse oder einer Asphäre vorgesehen. Diese Sammellinse oder ein entsprechendes Linsensystem ist insbesondere durch eine große Öffnung bei kurzer Brennweite gekennzeichnet. Unter großer Öffnung wird dabei eine Öffnung verstanden, die kleiner als 2,4 ist, während unter einer kurzen Brennweite eine solche in der Größenordnung von 10 mm verstanden wird. Bei Verwendung einer solchen Sammellinse oder eines entsprechenden Linsensystems werden auch die unter großem Winkel relativ zur optischen Achse vom Flaschenmund oder -gewinde abgegebenen Lichtstrahlen erfaßt. Zu diesem Zweck ist die Sammellinse oder das Linsensystem mit geringem Abstand oberhalb des Körpers bzw. des Flaschenmundes angeordnet. Bei genügend großem Durchmesser der Sammellinse kann diese gleichzeitig zur Erfassung derjenigen Lichtstrahlen dienen, die von den sich an den Flaschenmund anschließenden Bereichen wie Flaschenhals und Flaschenwand bis hinunter zum Flaschenboden ausgehen. In der Abbildung auf der lichtempfindlichen Schicht der Fernsehkamera oder der Diodenmatrix stellen dann diese aufeinanderfolgenden Bereiche konzentrisch zueinander angeordnete Kreisflächen bzw. Kreisringe dar. Anstelle einer Sammellinse kann man auch ein innenverspiegeltes Rohr verwenden, das koaxial zur Flasche angeordnet ist. Durchmesser und Länge dieses Rohres hängen davon ab, welche Bereiche der Flasche abgebildet werden sollen.

Ein weiterer Vorteil der neuen Anordnung ist darin zu sehen, daß bei Beibehaltung des gewählten konstruktiven Aufbaus auch Bereiche im Inneren des Körpers erfaßt werden können, sofern dieser eine entsprechende Öffnung aufweist, wie es insbesondere bei einer Flasche der Fall ist. In diesem Fall können also auch der Flaschenboden und darüber hinaus die Innenwand der Flasche kontrolliert werden.

d) Weitere Ausgestaltungen

Da die Abbildung des Flaschenbodens mit der genannten Sammellinse oder einem entsprechenden Linsensystem gleichzeitig mit dem Flaschenmund oder dem am Flaschenmund angeordneten Gewinde verhältnismäßig klein ausfällt, sind in der Praxis weitere Linsen zur Vergrößerung des Bildes des Flaschenbodens erforderlich, die zwischen der vorgesehenen Sammellinse und dem lichtempfindlichen elektronischen Bauteil oder innerhalb des innenverspiegelten Rohres anzuordnen sind. Hierbei kann man derart vorgehen, daß die Sammellinse eine entsprechende zentrische Bohrung aufweist, in und/oder über der eine oder weitere Linsen angeordnet sind. Mit Hilfe dieser Linsen wird die Pupille des Objektivs der Fernsehkamera oder der Diodenmatrix in den Flaschenhals verlagert, so daß die gesamte Innenwand des Körpers bzw. der Flasche einschließlich des Bodenbereiches erfaßbar ist.

Hinsichtlich der Dimensionierung der Sammellinse oder eines entsprechenden Linsensystems geht man zweckmäßig derart vor, daß diese bzw. dieses eine große Öffnung bei kurzer Brennweite aufweist. Dadurch ist gewährleistet, daß das vom Flaschenmund und vom gegebenenfalls vorhandenen Gewinde unter verhältnismäßig großem Winkel relativ zur optischen Achse abgestrahlte Licht aufgenommen wird. Sofern zwischen der Sammellinse und dem Kameraobjektiv eine weitere Linse angeordnet ist, sollte diese eine kleine Öffnung aufweisen. Hierunter werden Öffnungen verstanden, deren Kennziffer mehr als 16 beträgt. Bei Verwendung derartiger Linsen wird eine große Tiefenschärfe gewährleistet.

Sofern zwischen der Sammellinse und dem Kameraobjektiv mehrere Linsen vorgesehen sind, sollten diese einen Pupillenversetzer bilden, der zur Erzielung einer großen Tiefenschärfe und zur weitgehenden Abbildung des Behälterinneren ein entsprechend kleines Pupillenbild in der Behälteröffnung entwirft.

In allen Fällen empfiehlt es sich im übrigen, die Fernsehkamera bzw. die Diodenmatrix mit einem Weitwinkelobjektiv auszustatten.

e) Ausführungsbeispiele

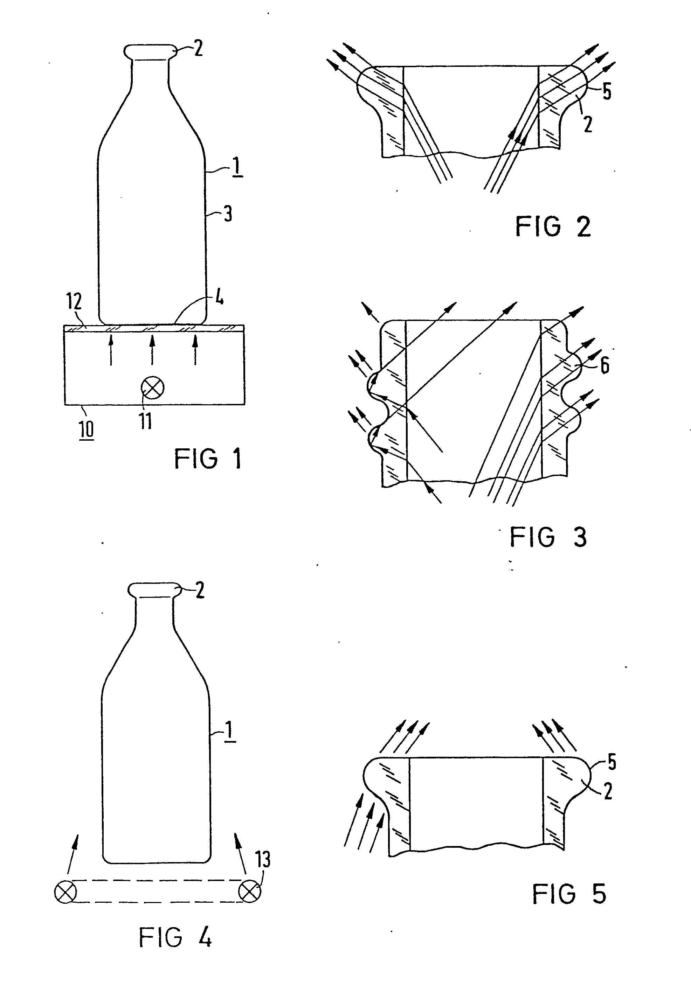

Ausführungsbeispiele der neuen Anordnung sind in den Figuren 1 bis 8 dargestellt. Anhand dieser Ausführungsbeispiele wird die Erfindung näher erläutert.

Fig. 1 zeigt eine Glasflasche 1 mit den für die Flascheninspektion wesentlichen Bereichen Flaschenmund 2, Flaschenwand 3 und Flaschenboden 4. Unterhalb der Glasflasche ist eine Beleuchtungseinrichtung 10 angeordnet, die im wesentlichen eine Lichtquelle 11 enthält und nach oben hin mit einer Streuglasscheibe 12 abgedeckt ist. Anstelle dieser Beleuchtungseinrichtung kann beispielsweise auch ein Lichtrohr verwendet werden, wie es in der älteren Patentanmeldung P 30 29 678.7 beschrieben ist.

Bei gleichmäßiger Ausleuchtung des Flaschenbodens treten an dem gegebenenfalls mit einem Gewinde 6 versehenen Flaschenmund 2 die Lichtstrahlen in der in den Figuren 1a und 1b dargestellten Weise aus der Glasoberfläche aus. Dabei ist gemäß der Darstellung in Fig. 1a der Flaschenmund außen mit einem Lichtkranz umgeben, während die Dichtlippe selbst dunkel erscheint. Bei der Beobachtung des Flaschenmundes zeigen sich eventuell vorhandene Beschädigungen dadurch, daß der Lichtkranz unterbrochen ist oder daß im Bereich der Dichtlippe Reflexe von Bruchstellen auftreten. Bei der Inspektion eines gemäß Fig. 1b mit einem Gewinde versehenen Flaschenmundes zeigen sich die Windungen des Schraubverschlusses mit hellen Streifen belegt, die einen guten Kontrast zu den übrigen Bereichen des Gewindes und des Mundes aufweisen. Betrachtet man den Flaschenmund von innen durch die Flaschenöffnung hindurch, so liegen die erwähnten hellen Streifen etwas höher als bei einer Betrachtung von außen. Im übrigen geht bei

stetig abnehmendem Blickwinkel relativ zur optischen Achse
der von außen sichtbare Streifen in zwei eng benachbarte
Streifen über, die gleichzeitig in der Intensität schwächer
werden und schließlich verschwinden.

Gemäß der Darstellung in Fig. 2 kann man die Flasche auch
von unten mit einer ringförmigen Lichtquelle beleuchten,
deren Durchmesser größer oder wesentlich größer als der
Außendurchmesser der Flasche ist. Die Lichtstrahlen nehmen
dann den in Fig. 2a dargestellten Verlauf an, so daß
die Dichtlippe gleichmäßig hell erscheint, während der
sie umgebende Lichtkranz hier fehlt. Flaschen mit einem
Schraubverschluß gemäß Fig. 1b zeigen bei dieser Art der
Beleuchtung nur eine geringfügige Verschiebung und Verbreiterung der von innen und außen auf den Windungen
sichtbaren hellen Streifen. Eine Kombination der Beleuchtung gemäß Fig. 1 und Fig. 2 ist möglich unter Verwendung
einer kreisförmigen Leuchtfläche, deren Durchmesser größer
als der Flaschendurchmesser ist. Hierzu wird die Beleuchtungseinrichtung gemäß Fig. 1 zweckmäßig mit einer Streuglasscheibe 12 versehen, die zwischen der Lichtquelle und
der Flasche angeordnet ist.

Zur Inspektion des Flaschenmundes wird gemäß Fig. 3 beispielsweise eine Fernsehkamera 20 verwendet, die mit einem
Objektiv 21 ausgerüstet ist. Hierbei ist unmittelbar oberhalb des Flaschenmundes 2 die Sammellinse 22 angeordnet,
mit der die von dem Flaschenmund ausgehenden Lichtstrahlen
in das Objektiv der Fernsehkamera projiziert werden. Bei
der verwendeten Sammellinse kann es sich beispielsweise
um eine Fresnel-Linse oder eine Asphäre oder auch um einen
Tripel-Kondensor handeln. Weiterhin kann anstelle einer
Fernsehkamera eine Diodenmatrix zur Anwendung kommen.

Gemäß der Darstellung in Fig. 4 ist die in Fig. 3 gewählte Anordnung auch bei der Inspektion von Flaschen zu verwenden, deren Mund 2 mit einem Gewinde 6 versehen ist.

Bei dieser Art der Flascheninspektion bildet sich der Dichtlippenrand als heller Ring ab, während das Gewinde als helle Spirale erscheint.

Die optische Anordnung gemäß Fig. 3 und 4 ist weiterhin dadurch gekennzeichnet, daß die Pupille des Kameraobjektivs 21 durch die Sammellinse 22 oder durch ein entsprechendes Linsensystem in das Innere des Flaschenhalses abgebildet wird. Bei Wahl einer entsprechend kleinen Blende kann eine hinreichend große Tiefenschärfe erzielt werden, so daß Flaschenmund, Flascheninnenwand und Flaschenboden gleichzeitig scharf auf den Sensor der Fernsehkamera 20 abgebildet werden. Die einzelnen Punkte auf den Prüfbereichen der Flaschen werden jeweils nur über bestimmte Zonen der Sammellinse 22 abgebildet. Die abbildenden Zonen liegen für den Flaschenmund oder das Gewinde in den äußeren Bereichen und für das Flascheninnere (Flaschenboden und Flascheninnenwand) näher an der optischen Achse. Dabei entsteht zwischen der Sammellinse 22 und dem Kameraobjektiv 21 eine Zwischenabbildung des Flaschenbodens und der Flascheninnenwand. Ein Zwischenbild des Flaschenmundes und des Gewindes entsteht hierbei jedoch nicht.

Gemäß Fig. 5 besteht auch die Möglichkeit, mit einem geeigneten Weitwinkelobjektiv 23 das Gewinde 6 eines Flaschenmundes von innen aufzunehmen. Im Unterschied zu den Anordnungen gemäß Fig. 3 und Fig. 4 wird bei der Anordnung gemäß Fig. 5 die Pupille des Kameraobjektivs 21 durch die Sammellinse 22 dicht oberhalb des Flaschenmundes abgebildet. Außerdem entsteht für alle Prüfbereiche der Flasche eine Zwischenabbildung zwischen der Linse 23 und dem Kameraobjektiv 21.

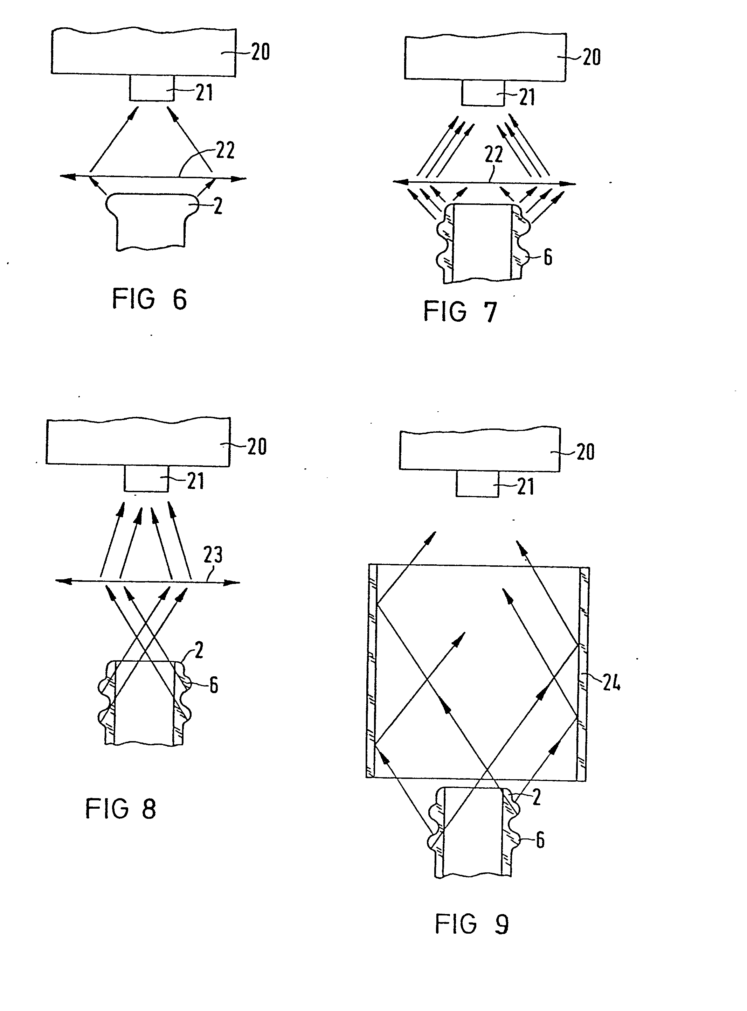

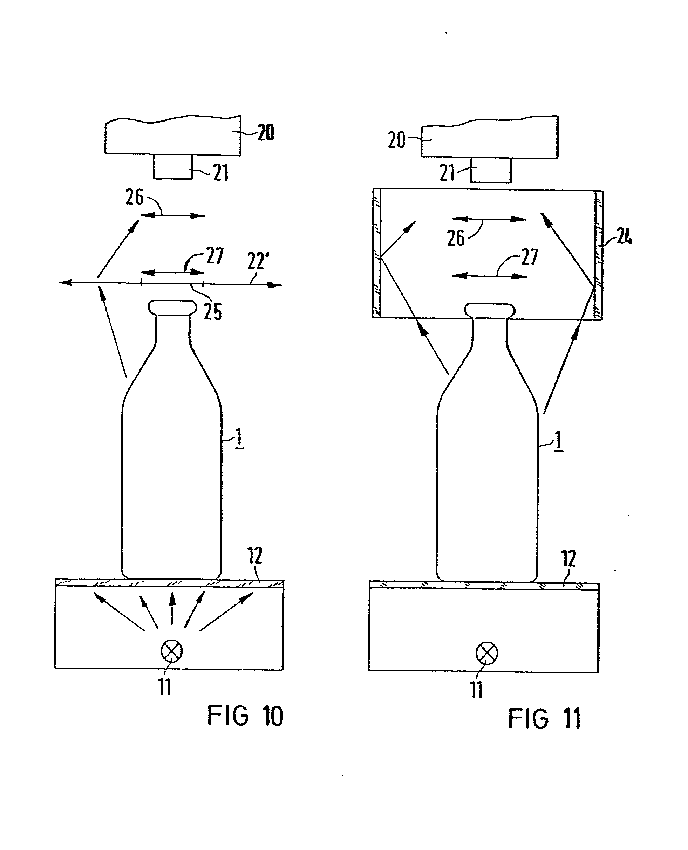

Die Abbildung des Flaschenmundes auf der lichtempfindlichen Schicht einer Fernsehkamera läßt sich gemäß der Darstellung in Fig. 6 auch mit Hilfe eines innenverspiegelten Rohres 24 vornehmen, dessen Durchmesser größer bzw.

wesentlich größer als der Außendurchmesser des Flaschenmundes ist. Eine solche optische Hilfseinrichtung ermöglicht darüber hinaus, das Gewinde 6 des Flaschenmundes
sowohl von innen als auch von außen abzubilden. Die dabei
in der Abbildung entstehenden Spiralen verlaufen konzentrisch zueinander mit gegenläufigem Windungssinn.

Die in Fig. 7 dargestellte Anordnung ist dazu geeignet,
den gesamten interessierenden Bereich einer Flasche zu
inspizieren. Hierzu ist entsprechend Fig. 1 unterhalb der
Flasche die Beleuchtungseinrichtung 10 mit der Lichtquelle
11 und der Streuglasscheibe 12 angeordnet. Oberhalb der
Flasche befindet sich in deren optischer Achse die Fernsehkamera 20 mit dem Objektiv 21. Unmittelbar über dem
Flaschenmund ist die Sammellinse 22′ angeordnet, die im
vorliegenden Fall mit einer zentrischen Bohrung 25 versehen ist. Dieser zentrischen Bohrung sind die beiden Linsen 26 und 27 zugeordnet, mit denen die Pupille des Objektivs 22 in den Flaschenhals verlagert wird. Die zentrische Bohrung 25 in der Sammellinse 22′ kann entfallen, wenn die Linse 27 eine entsprechend kleinere Brechkraft aufweist. Dann bilden die Linsen 26 und 27 zusammen
mit der inneren Zone der Sammellinse 22′ die Pupille des
Kameraobjektivs 21 in den Flaschenhals ab.

Auf diese Weise kann eine Inspektion der Innenwandung der
Flasche sowie des Flaschenbodens erfolgen. Der Durchmesser der Sammellinse 22′ ist derart gewählt, daß auch die
von der Außenwand der Flasche ausgehenden Lichtstrahlen
auf die lichtempfindliche Schicht der Fernsehkamera 20
projiziert werden.

Die in Fig. 8 dargestellte Anordnung weicht dadurch von
der Anordnung gemäß Fig. 7 ab, daß anstelle der Sammellinse 22′ ein innenverspiegeltes Rohr 24′ verwendet ist.
Der Durchmesser dieses Rohres und seine Länge sind derart gewählt, daß auch die von der Außenwand der Flasche

**0060918**

ausgehenden Lichtstrahlen von der Fernsehkamera 20/21 erfaßt werden.

Eine Inspektion des gesamten Flaschenbereiches kann auch dadurch erfolgen, daß man dem Flaschenmund ein innenverspiegeltes Rohr mit kleinem Durchmesser, der äußeren Flaschenwand ein dazu konzentrisch angeordnetes innenverspiegeltes Rohr mit großem Durchmesser und dem Flaschenboden und der Flascheninnenwand eine Linse zuordnet, über die der jeweilige Flaschenbereich auf die fotoelektrische Schicht projiziert wird.

Die der Fernsehkamera nachgeschaltete Auswerteelektronik ist nicht Gegenstand der vorliegenden Anmeldung. Entsprechende elektronische Auswerteeinrichtungen sind aus dem Stand der Technik bekannt und beispielsweise der DE-OS 28 48 316 und der DE-OS 29 16 361 zu entnehmen.

8 Figuren
9 Patentansprüche

0060918

Patentansprüche

1. Anordnung zur Erfassung der Oberflächenstruktur eines rotationssymmetrischen Körpers, insbesondere zur Erfassung von Beschädigungen an einem Flaschenmund, bestehend aus einer Beleuchtungseinrichtung, aus einer optischen Einrichtung, welche die von dem Körper ausgehenden Lichtstrahlen um- oder ablenkt, und aus einem lichtempfindlichen elektronischen Bauteil, auf das die von dem Körper ausgehenden Lichtstrahlen mittels der optischen Einrichtung projiziert werden, sowie aus einer dem lichtempfindlichen elektronischen Bauteil nachgeordneten elektronischen Einrichtung zur Auswertung elektrischer Bildsignale, d a d u r c h  g e k e n n z e i c h n e t , daß die Beleuchtungseinrichtung (11, 13) auf der dem lichtempfindlichen elektrischen Bauteil (20) abgekehrten Seite des rotationssymmetrischen Körpers (1) angeordnet ist und daß die optische Einrichtung aus einer Sammellinse (Fresnel-Linse, Asphäre) (22, 23) oder aus einem innenverspiegelten Rohr (24) besteht, deren bzw. dessen Durchmesser wesentlich größer ist als der Durchmesser des Körpers.

2. Anordnung nach Anspruch 1, d a d u r c h  g e - k e n n z e i c h n e t , daß zwischen der Sammellinse (22) und dem lichtempfindlichen elektronischen Bauteil (20) oder innerhalb des innenverspiegelten Rohres (24) eine oder mehrere weitere optische Linsen (26, 27) angeordnet sind.

3. Anordnung nach Anspruch 2, d a d u r c h  g e - k e n n z e i c h n e t , daß die weitere Linse (26, 27) oder die weiteren Linsen in und/oder über einer zentrischen Bohrung (25) der Sammellinse (22') angeordnet sind.

4. Anordnung nach einem der Ansprüche 1 bis 3, d a - d u r c h  g e k e n n z e i c h n e t , daß die 'Sammellinse oder das Linsensystem eine große Öffnung bei kurzer Brennweite aufweist.

5. Anordnung nach einem der Ansprüche 1 bis 4, d a -
d u r c h   g e k e n n z e i c h n e t , daß die weitere Linse eine kleine Öffnung aufweist.

6. Anordnung nach einem der Ansprüche 1 bis 4, d a -
d u r c h   g e k e n n z e i c h n e t , daß die weiteren Linsen einen Pupillenversetzer bilden.

7. Anordnung nach einem der Ansprüche 1 bis 6, d a -
d u r c h   g e k e n n z e i c h n e t , daß die Fernsehkamera bzw. die Diodenmatrix (20) mit einem Weitwinkelobjektiv (21) ausgestattet ist.

8. Anordnung nach einem der Ansprüche 1 bis 7, d a -
d u r c h   g e k e n n z e i c h n e t , daß die Beleuchtungseinrichtung (10) eine Streuglasscheibe (12) enthält, die zwischen der Lichtquelle (11) und dem Behälter
(1) angeordnet ist.

9. Anordnung nach Anspruch 1 mit einem innenverspiegelten
Rohr, d a d u r c h   g e k e n n z e i c h n e t ,
daß konzentrisch zu dem Rohr ein zweites innenverspiegeltes Rohr angeordnet ist, dessen Durchmesser größer als
der Durchmesser des erstgenannten Rohres ist.

0060918

FIG 1

FIG 2

FIG 3

FIG 4

FIG 5

0060918

FIG 6

FIG 7

FIG 8

FIG 9

FIG 10

FIG 11

# 0060918

Nummer der Anmeldung

**Europäisches Patentamt**

## EUROPÄISCHER RECHERCHENBERICHT

EP 81 10 8232

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A,D | DE-A-2 718 802 (PONNDORF H.G.) <br> * Seiten 5,7; Figur 1 * | 1 | G 01 N 21/90 |
| A | PATENTS ABSTRACTS OF JAPAN, Band 4, Nr. 58(P-9)(540), 30. April 1980, Seite 14P9 <br> & JP - A - 55 27918 (HITACHI ZOSEN K.K.) 28-02-1980 * Insgesamt * | 1,9 | |
| A | US-A-3 770 969 (R. ANSEVIN et al.) <br> * Spalte 2, Figur 2 * | 1,5 | |
| A | US-A-4 083 637 (B. ELLINGER et al.) <br> * Spalte 2, Figur 1 * | 1,8 | |

**RECHERCHIERTE SACHGEBIETE (Int. Cl. ³)**

G 01 N 21/90

Der vorliegende Recherchenbericht wurde für alle Patentansprüche erstellt.

| Recherchenort <br> DEN HAAG | Abschlußdatum der Recherche <br> 26-06-1982 | Prüfer <br> BOEHM CH.E.D. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

 

& : Mitglied der gleichen Patentfamilie, übereinstimmendes Dokument

EPA Form 1503. 03.82